# EUROPEAN PATENT APPLICATION

(11) **EP 4 151 407 A1**
(43) Date of publication of application: **22.03.2023**
(21) Application number: 21855863.3
(22) Date of filing: 26.07.2021
(51) Int. Cl.: B33Y 10/00, B33Y 80/00, B29C 64/153, A61F 5/02, A61F 2/60

(54) **BODY-MOUNTED COMPONENT, AND METHOD FOR MANUFACTURING SAME**

(30) Priority: 12.08.2020 JP 2020136102
(71) Applicant: KONICA MINOLTA, INC., Tokyo 100-7015 (JP)
(72) Inventor: WASHIZU, Takashi, Tokyo 100-7015 (JP); ISHIGAI, Takuya, Tokyo 100-7015 (JP)
(74) Representative: Gille Hrabal Partnerschaftsgesellschaft mbB Patentanwälte
(86) International application number: PCT/JP2021/027573
(87) International publication number: WO 2022/034784

(57) **Abstract**

The present invention addresses the problem of providing a body-mounted component, molded using a 3D printer, which has excellent flexibility, with which the occurrence of breakages and the like when bent is suppressed, and which has a comfortable fit, and a method for manufacturing the same. A body-mounted component according to the present invention is mounted around a joint of the body, and is characterized in that the body-mounted component is formed by means of a resin material for a 3D printer, and in that the tensile elongation at fracture of the resin material is at least equal to 200%. Further, the method for manufacturing the same is characterized by including: a step 1 of forming a powder of the resin material for the 3D printer into a thin layer; a step 2 of selectively shining laser light onto the formed thin layer to form a molded object layer obtained by sintering or melt-bonding resin particles contained in the powder; and a step 3 of laminating the molded object layers by repeating step 1 of forming the thin layer and step 2 of forming the molded object layer a plurality of times, in this order.

## Description

### TECHNICAL FIELD

The present invention relates to a body-mounted component and a method for manufacturing the same, and more specifically, to a body-mounted component molded by a 3D printer having excellent flexibility, suppressing the occurrence of breakage when bent, and having comfortable wearability, and a method for manufacturing the same.

### BACKGROUND ART

As body-mounted components such as body trunk orthoses and hand joint orthoses (hereinafter simply referred to as "orthoses"), a method of utilizing a 3D printer that can relatively easily produce a three-dimensional molded object with a complicated shape has been increasing. In other words, using resin materials, various 3D printing methods such as thermal melt extrusion, powder sintering lamination or powder bed melt bonding have been increasingly applied to the manufacturing of orthoses.

In these examples, the basic function of the orthosis is to fix the orthosis to the body, so the elastic modulus of the molded object is important. However, around the joints of the body, it is important for the orthosis to have appropriate flexibility in order to achieve comfort and better walking. As a result, there was a problem that the rupture elongation characteristics of the molded object were insufficient and the molded object was damaged at the bent portion.

Patent Document 1 discloses an example of an orthosis that supports a joint portion in a movable manner, in which a hard material and a soft material are integrally formed using a 3D printer. However, in the technique disclosed in Patent Document 1, although flexibility is ensured by forming the joints with a soft material, it is an orthosis that is insufficient to properly fix the joints. It does not meet the requirements for an orthosis that can properly fix joints and can also achieve flexibility due to its elongation.

### CITATION LIST

### Patent Literature

Patent Document 1: JP-A 2015-53950

### SUMMARY OF THE INVENTION

### TECHNICAL PROBLEM

The present invention was made in view of the above-mentioned problems and circumstances, and the problem to be solved is to provide a body-mounted component molded by a 3D printer having excellent flexibility, suppressing the occurrence of breakage when bent, and having comfortable wearability, and a method for manufacturing the same.

### SOLUTION TO PROBLEM

In the process of examining the causes of the above-mentioned problems, the present inventor has found that, by forming the body-mounted component from a resin material for a 3D printer and by setting the tensile rupture elongation of the resin material to a specific value or higher, a body-mounted component having excellent flexibility, suppressing the occurrence of breakage when the component is bent, and having a comfortable wearability may be obtained.

In other words, the above problem for the present invention is solved by the following means.
1. A body-mounted component to be worn around a joint of a body, wherein the body-mounted component is formed from a resin material for a 3D printer; and a tensile rupture elongation of the resin material is 200% or more.
2. The body-mounted component according to item 1, wherein a tensile modulus of the resin material is 800 MPa or more.
3.The body-mounted component according to item 1 or 2, wherein the tensile rupture elongation is 400% or more, and the tensile modulus is 900 MPa or more.
4. The body-mounted component according to any one of items 1 to 3, wherein the resin material is polypropylene.
5. The body-mounted component according to any one of items 1 to 4, wherein the resin material contains talc as an additive in the range of 1 to 5 mass%.
6. A method for manufacturing a body-mounted component according to any one of items 1 to 5, comprising:
   a process 1 of forming a thin layer of a powder of the resin material for a 3D printer;
   a process 2 of selectively irradiating a laser beam onto the formed thin layer to form a molded object layer in which resin particles contained in the powder are sintered or melt-bonded; and
   a process 3 of repeating the process 1 of forming the thin layer and the process 2 of forming the molded object layer in this order a plurality of times to laminate the molded object layers.

### ADVANTAGEOUS EFFECTS OF INVENTION

By the above means of the present invention, it is possible to provide a body-mounted component molded by a 3D printer and a manufacturing method thereof, which has excellent flexibility, suppresses occurrence of breakage when bent, and has comfortable wearability.

Although the expression mechanism or action mechanism of the effect of the present invention has not been clarified, it is inferred as follows.

In conventional body-mounted components, the basic function of the body-mounted components is only to fix them to the body, and for this reason the elastic modulus of the molded object has been emphasized. However, body-mounted components fabricated by a 3D printer lack the rupture elongation property of the fabricated parts and break at bending parts.

The body-mounted component of the present invention is a body-mounted component to be worn around a joint of the body, the body-mounted component being formed by a resin material for a 3D printer, and characterized in that the tensile rupture elongation of the resin material is 2 00% or more. Furthermore, it is a preferred embodiment from the viewpoint of obtaining the effect of the present invention that the tensile modulus of the resin material is 800 MPa or more.

By using a resin material having such physical properties to fabricate a body-mounted component using a 3D printer, the rupture elongation property of the molded object may be made sufficient, and a body-mounted component that does not break at a bend may be obtained. In addition, the body-mounted component of the present invention has sufficient rigidity for the part to be fixed due to its high elasticity modulus.

In addition, as mentioned above, for "orthoses that require bending", it important to stretch while being fixed. In the selection of resin material to achieve "elongation", it is possible to individually create and evaluate a molded object with a 3D printer, but this is a complicated and inefficient process.

The present inventors compared several materials in terms of the properties of injection-molded objects as a study of a simple evaluation method, and found that, with respect to a rupture elongation, the trends did not match between injection-molded objects and 3D-molded objects, making it difficult to predict.

The present inventors investigated this phenomenon, and found that the crystal states of inj ection-molded objects and 3D-molded objects were different in the same material as determined by X-ray diffraction (XRD). In the injection-molding process, the molten resin is "rapidly cooled" when it touches the mold, whereas in the 3D-molded process, the resin is "slowly cooled" in a pre-heated environment. This difference in cooling conditions was thought to be the cause of the different crystalline state. Therefore, when the injection-molded objects were reheated and then "slowly cooled" under conditions similar to those of the 3D-molded, tensile tests of these samples showed that even the injection-molded objects reproduced the elongation tendency similar to that of the 3D-molded object.

Furthermore, as a result of experiments on various materials, it was found that if the elongation at break is 200% or more after reheating and slow cooling of the injection-molded object, and preferably if the elastic modulus of the injection-molded object is 80 MPa or more, and it was found that the material could be applied to orthoses that require bending.

### BRIEF DESCRIPTION OF DRAWINGS

FIG. 1 is a diagram showing an example of a post-processing method performed by an orthotist during conventional manual manufacturing of an orthosis.
FIG. 2 is a diagram showing an example of the overall configuration of an orthosis manufacturing system in an embodiment.
FIG. 3A is a device for evaluating the rigidity of an orthosis and a graph showing an example of evaluation.
FIG. 3B is a device for evaluating the rigidity of an orthosis and a graph showing an example of evaluation.
FIG. 4 is a side view schematically showing a configuration of a three-dimensional molding apparatus according to an embodiment of the present invention.
FIG. 5 is a diagram showing a main part of a control system of a three-dimensional molding apparatus according to an embodiment of the present invention.

### DETAILED DESCRIPTION

The body-mounted component of the present invention is a body-mounted component to be worn around a joint of the body, the body-mounted component being formed by a resin material for a 3D printer, and characterized in that the tensile rupture elongation of the resin material is 2 00% or more. This feature is a technical feature common to or corresponding to the following embodiments.

As an embodiment of the present invention, from the viewpoint of expressing the effect of the present invention, it is preferable that the tensile modulus of the resin material is 800 MPa or more, in the point of making the orthosis lightweight by reducing the thickness and improving comfort when worn the orthosis.

When the tensile rupture elongation is 400% or more and the tensile modulus is 900 MPa or more, it is possible to provide a body-mounted component that is comfortable to wear by having excellent flexibility and bendability, and by reducing thickness and weight.

Polypropylene is a preferred resin material from the viewpoints of flexibility, bendability, and light weight.

It is preferred that the resin material contains talc as an additive in the range of 1 to 5 mass%, from the viewpoint of making it easier to control the balance between tensile rupture elongation and tensile modulus.

The method for making a body-mounted component of the present invention comprises a process 1 for forming a thin layer of a powder of a resin material for a 3D printer, and a process 2 of selectively irradiating the formed thin layer with a laser beam to form a molded object layer in which resin particles contained in the powder are sintered or melt-bonded, and a process 3 in which the process 1 of forming the thin layer and the process 2 of forming the molded object layer are repeated a plurality of times in this order to stack the molded object layers.

A detailed description of the present invention, its components, and the form and embodiment of carrying out the present invention will be given below. In this application, "to" is used in the sense of including the numerical values described before and after "to" as lower and upper limits.

### «Outline of the body-mounted component of the present invention»

The body-mounted component of the present invention is a body-mounted component to be worn around a joint of the body. It is characterized in that the body-mounted component is formed by a resin material for a 3D printer, and the tensile rupture elongation of the resin material is 200% or more.

As the "body-mounted components" of the present invention, "orthoses" are known to be worn on a part of the human body to correct, support or fix the part of the human body. Orthotics are mainly worn to compensate for a reduced function of a part of the human body or to protect or support the affected part by restricting the movement of a joint of the human body when the function of the part of the human body is reduced due to an illness or an injury.

This type of orthosis is generally worn on a part of the human body (hereinafter referred to as an "orthotic wearing part", for example, feet, elbows, wrists, legs and knees). On the other hand, unlike a cast made of gypsum, this type of orthosis is required to have a high degree of conformity to the patient's orthotic site in terms of shape and elasticity, because the orthotic site must be able to move freely to some extent while limiting the movement of the joints of the human body. In addition, because this type of orthosis is worn on a daily basis, comfort and strength (rigidity) are also required.

On the other hand, the completed form of each orthosis varies greatly from individual to individual because each patient's orthosis application site and the patient's physical disability (e.g., fracture condition) differ from patient to patient.

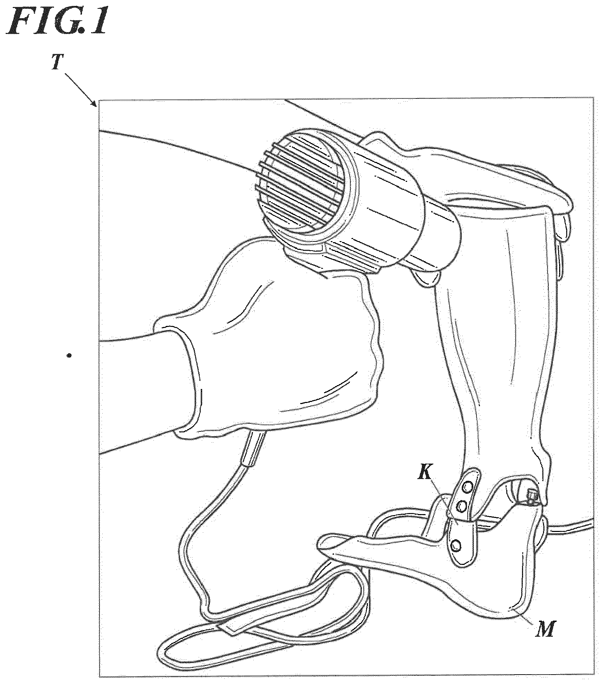

Conventionally, orthoses have been handmade by physicians and professional technicians (hereinafter referred to as "orthotists") to fit each patient. A specific example of a conventional manufacturing method for an ankle foot orthosis (AFO: Ankle Foot Orthosis) is a manual process as described in the following processes (1) to (7).
(1) Wrap a protective film around the patient's leg.
(2) Mark the palpation result on the protective film.
(3) A negative foot model is made using a cast.
(4) A positive foot model is made by pouring plaster into the negative foot model and allowing it to harden.
(5) Processing is performed based on the markings transferred onto the plaster.
(6) A thermoplastic resin sheet film is pressed against the positive foot model, and the sheet film is cured to form an orthotic device in accordance with the foot model.
(7) The orthotist or other person performs post-processing (e.g., shape adjustment using a heat gun as shown in FIG. 1) on the orthosis molded above to suit the patient, and the final orthosis is fabricated.

On the other hand, with the recent increase in accuracy of measuring devices and three-dimensional molding apparatuses, methods of three-dimensional molding of orthosis using a three-dimensional molding apparatus such as a 3D printer are being studied (for example, see JP-A 2013-530757).

In addition, in the conventional method based on processes (1) to (7) above, there is a problem that the quality of the orthosis is easily affected by the knowledge based on individual experience and technical differences of the orthotists, and that the quality of the device is easily varied. In addition, in the conventional method, the orthosis is designed or redesigned each time according to the specific symptoms of the patient, and is not manufactured using detailed design drawings. Therefore, it is difficult to manufacture an orthosis that is identical to the original orthosis, even if, for example, the orthosis is damaged or the patient wishes to have additional spares in case of damage or loss.

In view of such a problem, the shape (positional information in three-dimensional space) of an orthosis is scanned in 3D using a 3D scanner, and the digital data of the acquired three-dimensional shape is stored in a storage medium. While preserving the original shape of the orthosis, and by inputting such digital data into a three-dimensional molding apparatus such as a 3D printer and three-dimensionally molding the orthosis, an attempt is being made to manufacture (reproduce) an orthosis that is substantially the same as the original. It is expected that such a system will become popular in the future.

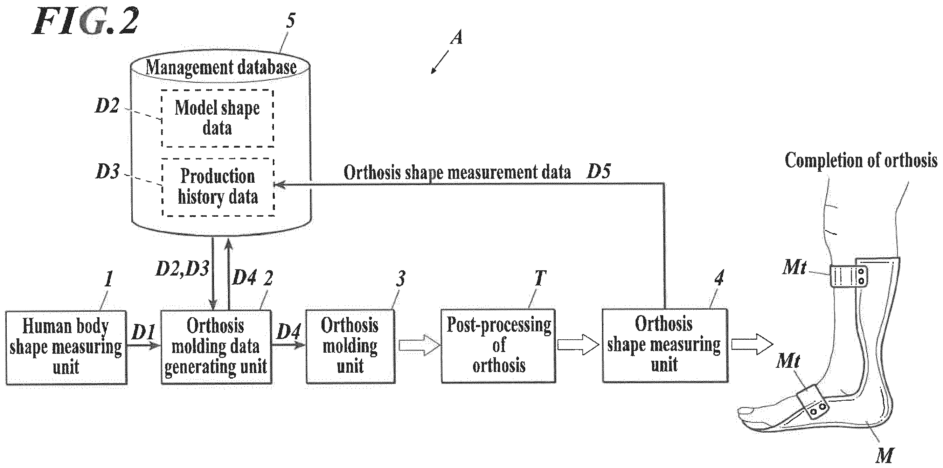

The system in question is briefly described with FIG. 2 as an illustration.

The orthotic manufacturing system A is equipped with a human body shape measurement unit 1, an orthosis molding data generating unit 2, an orthotic molding unit 3, an orthotic shape measurement unit 4, and a management database (DB) 5.

The human body shape measurement unit 1 measures the shape of an orthotic wearing part of the human body using a measuring device, and generates three-dimensional shape data D1 of the human body (hereinafter also referred to as "human body shape data").

The orthosis molding data generation unit 2 generates orthosis molding shape data D4 based on the human body shape data D1, orthosis molding shape data D2, and manufacturing history data D3 related to other orthosis manufactured in the past.

The orthotic molding section 3 is equipped with a three-dimensional molding apparatus such as a 3D printer, which will be described later, and three-dimensionally molds an orthotic device based on the molding shape data D4.

The orthotic device shape measurement unit 4 measures (3D scanning) the three-dimensional shape of the orthotic device by irradiating light onto the orthotic device and generates three-dimensional shape measurement data (hereinafter referred to as "orthotic device shape measurement data") D5 of the orthotic device based on the measurement results, and store the generated orthotic shape measurement data D5 in the management DB 5.

The management DB 5 accumulates molding shape data D2 that defines the base shape of the orthosis, manufacturing history data D3 that registers the shapes of orthoses manufactured in the past. The management DB 5 acquires orthotic shape measurement data D5 from the orthotic shape measurement unit 4 and stores corresponding orthotic human body shape data D1, and molding shape data D4 of the corresponding orthotic device, and stores them as manufacturing history data D3.

Note that the human body shape measurement unit 1, orthosis molding data generating unit 2, orthotic molding unit 3, orthotic shape measurement unit 4, and management DB 5 respectively have a computer composed of, for example, a CPU (Central Processing Unit (CPU), a ROM (Read Only Memory), a RAM (Random Access Memory), an operation input unit (keyboard, mouse), a display unit (liquid crystal display), an input port, and an output port. The functions of the human shape measurement section 1, the orthosis molding data generating unit 2, the orthotic molding section 3, the orthotic shape measurement section 4, and the management DB 5 are realized by the CPU referring to control programs and various data stored in ROM, and RAM.

In FIG. 2, the arrows indicate the manufacturing flow of the orthosis M (in the figure, Mt indicates a band with a fixture to secure the orthosis to the leg). In the manufacturing flow of the orthotic manufacturing system A, a process (indicated by T in FIG. 2) in which an orthotist manually post-processes the orthosis may be optionally added.

The present invention is directed to providing a body-mounted component fabricated by such a 3D printer that has excellent flexibility, suppresses breakage when flexed, and is comfortable to wear.

The term "flexibility" in the orthosis of the present invention indicates the ease of elastic deformation of a substance, and refers to the property of a substance to flexibly deflect by an external force. It indicates so-called flexibility, which is sometimes generally expressed as micro-elasticity.

Since the orthosis of the present invention has a function of fixing as well as flexibility, "rigidity" is also important. The term "rigidity" refers to the degree to which a material is resistant to dimensional change (deformation) in response to bending and twisting forces. When the deformation is small against the force, the rigidity is high (large), and when the deformation is large, the rigidity is low (small).

In orthoses, rigidity corresponding to the degree of tension of the lower limb is required after fitting with the shape of the user's lower limb. Furthermore, the shape must be suitable for the different walking movements of each user. For these reasons, the rigidity of the orthosis is adjusted according to the shape and thickness of the orthosis. However, since these lower limb shapes, tension levels, or walking movements are diverse, and the corresponding orthosis shapes are also diverse, it is important to quantitatively evaluate "rigidity" during design.

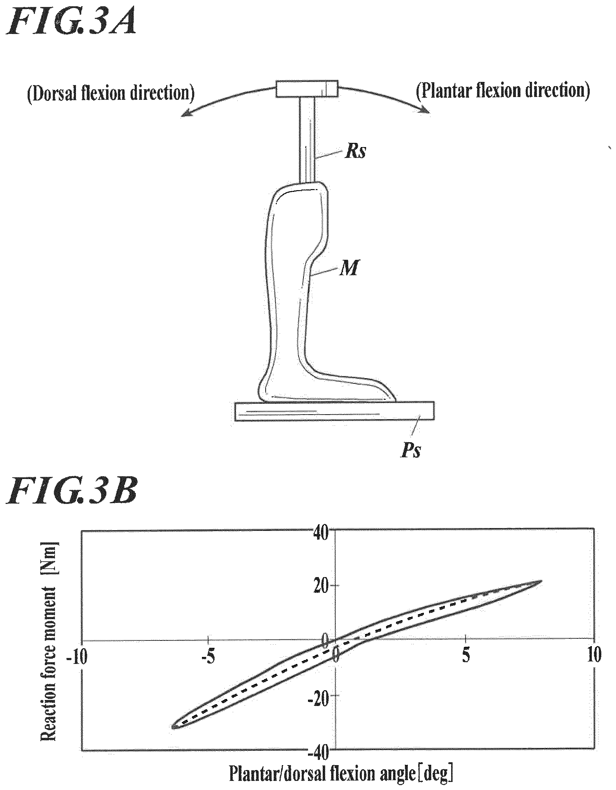

For example, in the case of the orthosis M worn on the lower limb as shown in FIG. 1, the plantar part is fixed to the plane stand Pa, and the lower leg of the orthosis is moved with the rotating shaft Rs, and a device capable of measuring the reaction force moment at each 1° angle in the plantar and dorsiflexion directions is used (see FIG. 3A).

The measurement data is obtained by measuring the reaction force moment at 1° intervals in the plantar and dorsiflexion directions, and the measurement angle range is, for example, from dorsiflexion 8° to plantar flexion 8°, assuming hemiplegic walking. The data are plotted on the coordinates of the reaction force moment [Nm] on the vertical axis and the angle of plantar and dorsiflexion [deg] on the horizontal axis to obtain a hysteresis curve. The slope of the approximate curve at an angle of 0° is used as the rigidity of the orthosis [Nm/deg] (see FIG. 3B).

It is also possible to determine a practical and comfortable orthotic thickness by making samples of the type of resin used in the orthotic and varying the thickness of the orthosis, and determining the thickness at which the "rigidity" becomes 1 Nm/deg.

The tensile rupture elongation (hereinafter also referred to as "elongation at break") and tensile modulus are preferably measured by the following method. It is preferable to use an injection-molded object with a controlled cooling condition as described above for a test sample. The "reheating and slow cooling conditions" may be set as desired, and the temperature rise may be in the range of -30 to 5 °C relative to the melting point, the cooling temperature may be in the range of 30 to 50 °C, and the cooling time may be in the range of 5 to 24 hours.

The test piece for evaluating the following elongation at break and tensile modulus are prepared as follows.

### <Example of Injection-molded object>

Powder the resin material pellets for a 3D printer with a hammer mill, jet mill, ball mill, impeller mill, cutter mill, pin mill, or biaxial crusher.

In addition to the above resin material powder, plate-shaped resin materials may also be crushed and used as crushed materials, or pellet-shaped resin materials may be used as they are, for the evaluation of characteristics in injection-molded objects.

Test piece shape: Adjust the shape to comply with JIS-K7161: 2014: 2014.

### [Injection molding conditions]

Mixing temperature: Melting point + 30 °C
Injection pressure: 10 MPa
Molding machine: Injection molding machine, manufactured by Leo Labs, Xplore Instruments Inc.
Reheat and slow cooling conditions: N₂ Oven, Raise the temperature to "the melting point -10 °C" and then cool down to 50 °C for 7 hrs.

### <Elongation at break>

The elongation at break is measured using, for example, a universal material testing machine TENSILON RTC-1250 (manufactured by A & D Co., Ltd.) to the above molded objects. The measurement conditions are set as follows. The distance to fracture is made as the elongation at break, which is evaluated based on the presence or absence of a yield point. (If the material is fractured before the yield point, it is assumed to have no yield point).

Test piece for tensile test: Shape to comply with JIS K7161: 2014: 2014: 2014
Tensile speed: 50 mm/min
Distance between chucks: 115 mm
Gauge distance: 75 mm

### <Tensile modulus>

The tensile modulus is measured by, for example, a universal material testing machine TENSILON RTC-1250 (manufactured by A&D Co., Ltd.). The measurement conditions are set as follows, and the tensile modulus is obtained by linear regression between 0.05 and 0.25% strain.

Test piece for tensile test: Shape to comply with JIS K7161: 2014: 2014
Tensile speed: 1 mm/min
Distance between chucks: 115 mm
Gauge distance: 75 mm

The components of the present invention are described in detail below.

### [1] Thermoplastic resin

A thermoplastic resin is more suitably used as a resin material for a 3D printer to form the orthosis of the present invention. Examples of the thermoplastic resin include a polypropylene resin, a polyethylene resin, a polyvinyl chloride resin, a polycarbonate resin, an ABS resin, a polyamide resin (especially, Nylon 6, Nylon 11, Nylon 12), an acrylic resin, a urethane resin, and an urethane-acrylic resin. Thermoplastic resins are lightweight and strong, and in addition, have good biocompatibility. In addition, the powder sintering lamination type three-dimensional molding is possible by using thermoplastic resin powder (hereinafter referred to as "resin powder").

A UV-curable resin or a thermosetting resin may be used as a material constituting the orthosis in place of the thermoplastic resin described above. As the UV-curable or thermosetting resins, for example, a polyurethane resin, an epoxy resin, a silicone resin, and an acrylic resin are useful.

The "crystalline thermoplastic resin", which is a preferred thermoplastic resin for the present invention, will be described in detail below.

### [Crystalline thermoplastic resin]

In the resin powder used in the present invention, it is preferable that the resin particles constituting the resin powder contain a crystalline thermoplastic resin as a main component. The main component referred to in the present invention is a composition in which 60 mass% or more of the total mass of the resin is a crystalline thermoplastic resin, preferably 80 mass% or more is a crystalline thermoplastic resin, and more preferably 90 mass% or more of the total mass of the resin is a crystalline thermoplastic resin, and particularly preferably a configuration in which all of the resin components are crystalline thermoplastic resins.

The crystalline thermoplastic resin applicable to the present invention has no particular limitation and may be selected according to the purpose. Examples thereof include polyolefin, polyamide, polyester, polyarylketone, polyphenylene sulfide, polyacetal, and fluororesin polymer. One of these may be used alone, or two or more may be used in combination.

The polyolefins mentioned above include, for example, polyethylene and polypropylene. One of these may be used alone, or two or more may be used in combination.

Among these, polypropylene is a preferred resin material because it is easy to control the balance between the tensile rupture elongation and the tensile modulus, and because it is flexible, bendable, and lightweight as an orthosis.

Examples of the polypropylene used in the present invention include a propylene homopolymer, a propylene-olefin copolymer (e.g., a copolymer of propylene and ethylene or α-olefin having 4 to 20 carbon atoms), block polypropylene, and a blended resin. Of these, block polypropylene is preferred because of its ease of obtaining mechanical strength.

Examples of the olefin used for copolymerization with propylene in the propylene-olefin copolymer include 1-butene, 1-pentene, 4-methyl-1-pentene, 1-hexene, 4-methyl-1-hexene, 1-heptene, 1-octene, 1-nonene, 1-dodecene, 1-tetradecene, 1-octadecene, and 1-eicosene. These may be used alone or in combination. Among these, ethylene and α-olefins having a carbon number of 4 to 10 are preferred.

An example of a method for producing polypropylene is a method of modifying polypropylene having an acid value of 0 mg KOH/g before modification (hereinafter referred to as "unmodified polypropylene") by graft polymerization with maleic anhydride. Further, a method of acid modification by copolymerizing propylene with acrylic acid, methacrylic acid or maleic anhydride may be mentioned.

As an unmodified polypropylene, for example, polypropylene homopolymer, ethylene-propylene random copolymer, ethylene-propylene block copolymer, ethylene-α-propylene copolymer, ethylene-α-propylene copolymer, propylene-α-propylene copolymer may be used.

The acid number of polypropylene may be controlled to a desired value by adjusting the addition ratio of acid monomers such as maleic anhydride, acrylic acid and methacrylic acid used in the above graft polymerization and copolymerization to unmodified polypropylene.

The acid number of polypropylene is preferably in the range of 1 to 41 mg KOH/g from the viewpoint of adhesion during molding, and the number average molecular weight of polypropylene is preferably in the range of 1000 to 10000 from the viewpoint of fracture properties, elastic modulus and rigidity of the molded product.

These are also available commercially. For example, as polyethylene, Novatic HDHJ580N manufactured by Nippon Polyethylene Co., Ltd., as polypropylene, NOBREN FLX80E4 manufactured by Sumitomo Chemical Co., Ltd., or CM646V manufactured by SunAllomer Ltd. may be used.

Examples of the polyamide include polyamide 410 (PA410), polyamide 6 (PA6), polyamide 66 (PA66, melting point: 265 °C), polyamide 610 (PA610), polyamide 612 (PA612), polyamide 11 (PA11), polyamide 12 (PA12); semi-aromatic polyamide 4T (PA4T), polyamide MXD 6 (PAMXD6), polyamide 6T (PA6T), polyamide 9T (PA9T, melting point: 300 °C), and polyamide 10T (PA10T). One of these may be used alone, or two or more may be used in combination. Among these, PA9T is also called as polynonamethylene terephthalamide, and it is composed of a diamine with nine carbons and a terephthalic acid monomer. It is called semi-aromatic because the carboxylic acid side is aromatic. Furthermore, the polyamides of the present invention also include those called aramids, which are composed of p-phenylenediamine and terephthalic acid monomers as wholly aromatic compounds whose diamine side is also aromatic.

Examples of the polyesters include polyethylene terephthalate (PET, melting point: 260 °C), polybutylene terephthalate (PBT, melting point: 218 °C), polylactic acid (PLA). In order to impart heat resistance, a polyester partially having aromatics containing terephthalic acid or isophthalic acid may also be suitably used in the present invention. As commercially available products, for example, as polybutylene terephthalate, Novaduran 5010R3 manufactured by Mitsubishi Chemical Corp. Ltd. may be cited.

Examples of the aforementioned polyarylketone include polyetheretherketone (PEEK, melting point: 343 °C), polyetherketone (PEK), polyetherketoneketone (PEKK), polyaryletherketone (PAEK), polyetheretherketoneketone (PEEKK), and polyetherketone ether ketone ketone (PEKEKK). In addition to the aforementioned polyarylketones, any crystalline polymer such as polyacetal, polyimide and polyethersulphone may be used. A polymer having two melting point peaks such as PA9T may also be used.

In contrast to crystalline thermoplastic resins used in the present invention, examples of amorphous thermoplastic resin include PVC (polyvinyl chloride), PS (polystyrene), PMMA (polymethylmethacrylate), ABS (acrylonitrile-acrylate), PS (acrylonitrile-acrylate), m-PPE (modified polyphenylene ether), and PES/PEUS (polyethersulfone).

It is also preferable to use a material having a tensile modulus different from that of the resin material or a material having rigidity different from that of the resin material mainly used for the orthosis of the present invention. In this case, it is possible to appropriately select and use a plurality of different resins from the resins described above, or to use resins and metals different from those described above. Specifically, a carbon fiber reinforced resin (Carbon Fiber Reinforced Plastic: CFRP), a glass fiber reinforced plastic (Glass Fiber Reinforced Plastic: GFRP), light metals, or alloys containing light metals may be used as examples.

### [Resin powder]

The resin material for a 3D printer of the present invention is preferably made by converting the thermoplastic resin described above into a resin powder (also referred to as "resin particles").

### (Preparation method)

The thermoplastic resin particles of the present invention are, for example, made by pulverizing pellets of the above-mentioned crystalline thermoplastic resin by various pulverization methods while controlling the particle diameter and shape. For example, it is preferred that the thermoplastic resin particles are prepared by milling by a milling method such as a mechanical milling method or a wet milling method to produce a powder, and then using a particle spheronization means of each method and selecting the conditions for implementing the particle spheronization process as appropriate.

In the mechanical milling method, the prepared crystalline thermoplastic particles are mechanically milled to produce primary particles with the desired average particle size.

As an example of a mechanical milling method applicable to the present invention, resin particles may be prepared according to the following method.

The crystalline thermoplastic particles may be frozen and then milled, or may be milled at room temperature. The mechanical milling method may be performed by known equipment for milling thermoplastics. Examples of such equipment include a hammer mill, a jet mill, a ball mill, an impeller mill, a cutter mill, a pin mill and a twin-shaft crusher.

In the mechanical crushing method, the crystalline thermoplastic resin particles may fuse with each other due to frictional heat emitted from the crystalline thermoplastic resin particles during crushing, and particles with a desired particle diameter may not be obtained. Therefore, the method of cooling and embrittling the crystalline thermoplastic resin particles using liquid nitrogen, and then crushing the crystalline thermoplastic resin particles is preferred.

According to the mechanical milling method, the amount of solvent to the crystalline thermoplastic particles or the method or speed of milling may be adjusted appropriately to adjust the average particle size of the crystalline thermoplastic particles finally prepared to the desired range. The particle size obtained by pulverization is determined by the operating time of the equipment, which is preferably in the range of 5 to 45 hours.

In the wet milling method, a crystalline thermoplastic tree is dissolved in a solvent by heating and stirring, the resin solution obtained by the dissolution is cooled while stirring, and the resin slurry obtained by the cooling is milled by vacuum drying while stirring to produce particles having the desired average particle diameter. Specifically, the method described in JP-A 3-12428 may be cited.

It is preferable that the resin powder has a volume average particle diameter MV of 1 to 200 µm for the resin particles constituting the resin powder, and the ratio of the volume average particle diameter MV to the number average particle diameter MN (MV/MN) is 2.5 or more, and a static bulk density of 0.30 g/cm³ or more.

### <Volume average particle diameter>

In the present invention, the volume average particle diameter MV of the resin particles is preferably in the range of 1 to 200 µm, more preferably in the range of 10 to 150 µm, and even more preferably in the range of 20 to 100 µm.

### (Measurement of the volume average particle diameter MV of the resin particles)

The volume average particle diameter MV of the crystalline thermoplastic resin particles used in the present invention was determined using a particle size distribution measuring device (Microtrac MT3300EXII, manufactured by MicrotracBEL Corp.), and the refractive index of the resin particles was set to 1.5. The measurement procedure was as follows: 0.1 g of resin particles was mixed with 0.2 g of surfactant EMAL E-27C (made by Kao Corporation) and 30 mL of water, and ultrasonic dispersion treatment was carried out for 10 minutes according to a conventional method.

### <Ratio of volume average particle diameter MV to number average particle diameter MN (MV/MN)>

For the resin particles used in the present invention, it is preferable that the ratio of the volume average particle diameter MV of the resin particles described above to the number average particle diameter MN obtained by the following method (MV/MN) is preferably 2.5 or more, more preferably in the range of 2.5 to 4.0. The value is more preferably in the range of 2.6 to 3.5, and particularly preferably in the range of 2.7 to 3.0.

In the present invention, the number average particle diameter MN of the resin particles is not particularly limited as long as it satisfies the conditions specified above. It is preferably in the range of 5 to 100 µm, more preferably in the range of 10 to 75 µm, particularly preferably in the range of 20 to 50 µm.

### (Measurement of number average particle diameter MN of resin particles)

The number average particle diameter MN of the crystalline thermoplastic resin particles used in the present invention was determined using a particle size distribution measuring device (Microtrac MT3300EXII, MicrotracBEL Corp.), and the particle refractive index of the resin particles was set to 1.5. The measurement procedure was as follows: 0.1 g of resin particles was mixed with 0.2 g of surfactant EMAL E-27C (made by Kao Corporation) and 30 mL of water, and ultrasonic dispersion treatment was carried out for 10 minutes according to a conventional method.

### <Static bulk density>

It is preferable for the resin powder used in the present invention to have a static bulk density of 0.30 g/cm³ or more, more preferably in the range of 0.30 to 0.42 g/cm³ or more, and even more preferably in the range of 0.35 to 0.40 g/cm³.

### (Measuring method of static bulk density)

The static bulk density of the resin powder according to the present invention may be determined by the following method.

When a cubic cup is used as the measuring vessel, the minimum volume is made to 25 cm³. When a cylindrical cup is used, the minimum volume is made to 35 cm³. The powder is allowed to flow through the measuring device until the excess powder overflows into the cup that serves as the receiver. Carefully scrape off the excess powder from the top of the cup by smoothly moving the blade of a spatula in vertical contact with the top of the cup, keeping the spatula vertical to prevent compaction and overflow of the powder from the cup.

Remove all the sample from the side of the cup as well and measure the mass of the powder (m) to 0.1%.

Then, calculate the static bulk density (g/cm³) by the formula: m/V₀ (V₀ is the volume of the cup). By using three different measurement samples, determine the average of the three measurements and make it the static bulk density (g/cm³).

### <Number average particle size distribution>

In the present invention, a preferred embodiment is the case in which the resin particles having an average particle diameter in the range of 0.15 to 0.41 times the number average particle diameter MN of the resin particles are present in the number equal to or more than the number of particles having the number average particle diameter MN.

That is, when M₁ is the number of particles having a number average particle diameter MN and M₂ is the number of particles having an average particle diameter in the range of 0.15 to 0.41 times the number average particle diameter MN, then M₁/M₂ is preferably 0.5 or less.

This allows particles having an average particle diameter in the range of 0.15 to 0.41 times the number average particle diameter MN are placed between the particles having a number average particle diameter MN, and the voids are filled, thereby increasing rigidity while maintaining strength.

### [Spheronization treatment]

A typical method of particle spheronization treatment applicable to the present invention is to apply mechanical impact force. Examples thereof include mechanical impact milling machines such as a Kryptron system (manufactured by Kawasaki Heavy Industries, Ltd.) or a Turbo Mill (manufactured by Turbo Industries, Ltd.).

In addition, as in devices such as Mechanofusion System (manufactured by Hosokawa Micron Corporation) and Hybridization System (manufactured by Nara Machinery Co., Ltd.), for example, polypropylene resin particles are pressed against the inside of the casing by centrifugal force by means of blades that rotate at high speed. A method of applying a mechanical impact force to the polypropylene resin particles by means of compressive force or frictional force may be mentioned.

Meteor Rainbow made by Nippon Pneumatic Co., Ltd. may also be used as a hot air treatment.

The following is an example of the specific equipment and conditions of a typical particle spheronization treatment method.

### <Particle spheronization treatment by COMPOSI: Particle design device>

COMPOSI (registered trademark of Nippon Coke Company) has an impeller rotating at high speed and a fixed collision plate inside a vessel. The impeller disperses the powder and applies an impact force to the powder to spheronize the particles. COMPOSI MP5, CP15, and CP60, manufactured by Nippon Coke Company may be mentioned.

As for the conditions for spheronization, for example, particle size reduction may be performed by dispersion treatment with a charging amount of 100 to 10000 g, a processing time of 30 to 80 minutes, and a peripheral speed of 40 to 100 m/s.

### <Spheronization treatment by Hybridization system (NHS): Particle design device>

Hybridization system NHS (Nara Machinery Co., Ltd.) is a device to spheronize irregularly shaped particles in a dry process using a force mainly due to impact forces between particles while the raw material is dispersed in a high-speed air stream.

Specific devices includes the NHS-0, NHS-1, NHS-2 NHS-3, NHS-4, and NHS-5, manufactured by Nara Machinery Co., Ltd.

For example, when using the NHS-3, the treatment may be performed with a charging amount of 600 to 1600 g, a processing time of 1 to 30 minutes, and a peripheral speed of 50 to 100 m/s.

### <Spheronization treatment by Meteor Rainbow: Surface modification device of fine powder>

Meteor Rainbow MR Type (manufactured by Nippon Pneumatic Co., Ltd.) is a surface modifier that disperses and sprays plastic fine particles into hot air (processing temperature: up to 400 °C), allowing the particles to melt with the hot air, and the particle temperature immediately reaches the melting start temperature. The fused particles are spheronized by the surface tension of the particles themselves. It has features such as uniform spheronization of fine particles, less thermal degradation of materials due to instantaneous heating and cooling, and no granulation between particles because they are processed in a completely dispersed state.

Specific devices includes MR-2 unit, MR-10, MR-50, and MR-100 manufactured by Nippon Pneumatic Co., Ltd.

For example, when performing a spheronization treatment using Meteor Rainbow MR, the treatment may be performed within the ranges of a charging amount of 0.5 to 5 kg/hour, a hot air flow rate of 500 to 2000 L/minute, and a discharge temperature of 300 to 600 °C.

### [Other materials]

The resin powder for a 3D printer used in the present invention may further contain other materials, such as a laser absorbing material and a flow agent, to the extent that they do not significantly interfere with the dense packing of the resin particles when forming melt bonds and thin layers by laser irradiation as described below and do not significantly reduce the accuracy of the three-dimensional molding.

### <Laser absorber>

From the viewpoint of more efficiently converting the light energy of the laser into heat energy, the resin powder for a 3D printer may further include a laser absorber. The laser absorber may be any material that absorbs a laser of the wavelength to be used and emits heat. Examples of such laser absorbers include carbon powders, Nylon resin powders, pigments and dyes. These laser absorbers may be used alone, or in combination of two or more.

The amount of the laser absorber may be set appropriately within the range in which melting and bonding of the resin particles are facilitated, and for example, the amount of the laser absorber may be set to be 0 mass% or more and less than 3 mass% of the total mass of the resin powder for a 3D printer.

### <Flow agent>

From the viewpoint of further improving the flowing ability of the resin powder for a 3D printer and facilitating handling of the resin powder during fabrication of a three-dimensional molded object, the resin powder for a 3D printer may further contain a flow agent.

The flow agent may be a material having a low coefficient of friction and self-lubricating properties. Examples of such flow agents include silicon dioxide and boron nitride. These flow agents may be used alone or in combination of two or more. The resin powder for a 3D printer described above is less likely to be charged by the resin particles even when flowing ability is increased by incorporation of the flow agent, allowing the resin particles to be more densely packed when forming a thin film.

For example, the amount of the above-mentioned silicon dioxide added may be set appropriately within the range in which the fluidity of the resin powder for a 3D printer is improved and melt bonding of the above-mentioned resin particles is sufficiently generated. For example, it is preferable to set the amount of silicon dioxide to be more than 0.01 mas% and less than 1 mass% of the total mass of the resin powder for a 3D printer.

### <Additive other than Flow agent>

Examples of the additive other than the flow agent include talc, calcium carbonate, glass balloons, glass cut fibers, glass milled fibers, glass flakes, glass powders, silicon carbide, silicon nitride, gypsum, gypsum whiskers, calcined kaolin, carbon black, zinc oxide, antimony trioxide, zeolite, hydrotalcite, metal fibers, metal whiskers, metal powders, ceramic whiskers, graphite, and carbon fibers. The resin composition may contain only one of these, or may contain two or more of these. The added amount of these is preferably in the range of 1 to 10 mass%, and more preferably in the range of 1 to 5 mass%.

It is particularly preferred that the resin material of the present invention contains talc as an additive in the range of 1 to 5 mass% from the viewpoint of better controlling the balance between the tensile rupture elongation and the tensile modulus and improving the tensile modulus. In particular, it is more preferred to include talc in the range of 1.5 to 3 mass%.

The resin powder for a 3D printer used in the present invention may be produced by synthesizing the resin particles and stirring and mixing with materials other than the resin particles as necessary.

### [2] Body-mounted component and method of manufacturing the same

A body-mounted component (orthosis) of the present invention is a three-dimensional molded object formed by using a resin powder for a 3D printer, and is characterized in that it is a sintered or melted body of the resin powder for a 3D printer.

The body-mounted component (orthosis) of the present invention may be produced by the powder bed fusion bonding (PBF) method (see below) using the aforementioned resin powder for a 3D printer.

### [Production method of orthosis]

The production method of an orthosis of the present invention may be performed in the same way as the usual powder bed melt bonding method, except that the resin powder for a 3D printer described above is used.

It is preferable for the orthosis of the present invention to have sections of different rigidity from the viewpoint of achieving both fixation to the body and elongation, and it is preferred that the rigidity is adjusted by the structure, by thickness, or by a material having a different tensile modulus than the main resin material, by thickness adjustment (thinner wall), or by holes (lattice structure, or honeycomb structure) to be provided.

It is preferred that the body-mounted component of the present invention has a drive portion (e.g., the portion indicated by K in FIG. 1) from the viewpoint of obtaining an orthosis that suppresses the occurrence of breakage when bent.

Specifically, the powder bed melt bonding method, which is a preferred method of making the orthosis of the present invention, comprises a process (1) of forming a thin layer of the resin powder for the 3D printer, a process (2) of selectively irradiating a laser beam onto the preheated thin layer with a laser beam to form a molded object layer in which resin particles contained in the resin powder for the 3D printer are melt-bonded, and a process (3) of repeating the process (1) and the process (2) a plurality of times in this order to stack the molded object layers.

By the process (2), one of the molded object layers constituting the orthosis is formed, and by repeating the process (1) and the process (2) in the process (3), the next layer of the orthosis is stacked, and the final orthosis is fabricated. The method of making the orthosis of the present invention may further contain a process (4) of preheating the formed thin layer of the resin powder for a 3D printer at least prior to the process (2).

### <Process of forming a thin layer of a resin powder for a 3D printer (Process (1))>

In this process, a thin layer of the resin powder (resin particles) for a 3D printer is formed. For example, the above resin powder for a 3D printer supplied from the powder supply unit is laid flat on the molding stage by a recoater. The thin layer may be formed directly on the molding stage, or may be formed so as to contact the resin powder for a 3D printer that has already been spread or the molded object layer that has already been formed.

The thickness of the thin layer may be set in accordance with the thickness of the molded object layer. The thickness of the thin layer may be set as desired depending on the precision of the orthosis to be fabricated, but is usually in the range of 0.01 to 0.30 mm. By setting the thickness of the thin layer to 0.01 mm or more, it is possible to prevent the resin particles in the lower layer from melting and bonding or the molded object layer in the lower layer from re-melting due to laser irradiation for forming the next layer. By setting the thickness of the thin layer to 0.30 mm or less, it is possible to conduct the laser energy to the bottom of the thin layer, and the resin particles contained in the resin powder for a 3D printer constituting the thin layer may be sufficiently melt-bonded throughout the entire thickness direction.

From the above viewpoint, it is more preferred that the thickness of the thin layer is in the range of 0.01 to 0.10 mm. From the viewpoint of more fully melting and bonding the resin particles in the entire thickness direction of the thin layer and less likely to cause cracking between layers, it is preferred that the thickness of the thin layer is set so that the difference from the beam spot diameter of the laser described below is within 0.10 mm.

### <Process of forming a molded object layer in which resin particles contained in a resin powder for a 3D printer are melt-bonded (Process (2))>

In this process, a laser is selectively irradiated to the position where the molded object layer should be formed among the formed thin layers, and the resin particles at the irradiated position are melted and bonded together. As a result, adjacent resin particles melt together to form a molten bond, which becomes the molded object layer. At this time, the resin particles that have received the energy of the laser also melt-bonded with the already formed layer, so that adhesion between adjacent layers also occurs.

The wavelength of the laser is preferably set within the range where the wavelength corresponding to the energy required for vibration or rotation of the constituent molecules of the resin particles is absorbed. The difference between the wavelength of the laser and the wavelength at which the absorption rate is highest should be small. Since the resin may absorb light in various wavelength ranges, it is preferable to use a laser with a wide wavelength band such as a CO₂ laser. For example, the wavelength of the laser is preferably in the range of 0.8 to 12 µm.

For example, the power at the output of the laser is preferably set within the range in which the above resin particles are sufficiently melt-bonded at the scanning speed of the laser as described below, and specifically, the power may be set within the range of 10 to 100 W. From the viewpoint of lowering the energy of the laser, lowering the manufacturing cost, and simplifying the configuration of the manufacturing apparatus, it is preferable that the power at the output of the laser is 60 W or less, and more preferably, it is 40 W or less.

The scanning speed of the laser is preferably set within a range that does not increase the fabrication cost and does not excessively complicate the device configuration. Specifically, it is preferable to be in the range of 20000 mm/sec, more preferably in the range of 1000 to 18000 mm/sec, even more preferably in the range of 2000 to 15000 mm/sec. It is further preferable to be in the range of 3 to 80 mm/sec, more preferably in the range of 3 to 50 mm/sec.

The beam diameter of the laser may be set according to the precision of the orthosis to be fabricated.

### <Stacking the formed thin layer of the resin powder for a 3D printer (Process (3))>

In this process, the process (1) and the process (2) are repeated to stack the molded object layers formed by the process (2). By stacking the molded object layers, the desired orthosis is fabricated.

### <Process of preheating the formed thin layer of the resin powder for a 3D printer (Process (4))>

In this process, prior to the process (2), it is preferable to preheat the thin layer of the resin powder for a 3D printer. For example, a heater may be used to heat the surface of the thin layer (standby temperature) to a temperature higher than the melting point of the resin particles by 15 °C or less, preferably 5 °C or less.

### <Others>

From the viewpoint of preventing a decrease in the strength of the orthosis due to oxidation of the resin particles during melt bonding, it is preferred that at least the process (2) is performed under reduced pressure or in an inert gas atmosphere. It is preferred that the pressure at the time of pressure reduction is 10⁻² Pa or less, and it is more preferred that the pressure is 10⁻³ Pa or less.

Examples of the inert gas that may be used in the present invention include a nitrogen gas and noble gases. Of these inert gases, a nitrogen (N₂) gas, a helium (He) gas or an argon (Ar) gas is preferred in terms of ease of availability.

From the viewpoint of simplifying the fabrication process, it is preferable to perform all of the processes (1) to (3) (or all of the processes (1) to (4) when process (4) is included) under reduced pressure or in an inert gas atmosphere.

### <Three-dimensional molding apparatus>

The tree-dimensional molding apparatus used in the present invention may be configured in the same way as known devices for making orthoses by the powder bed melt bonding method, except that the resin powder for a 3D printer described above is used.

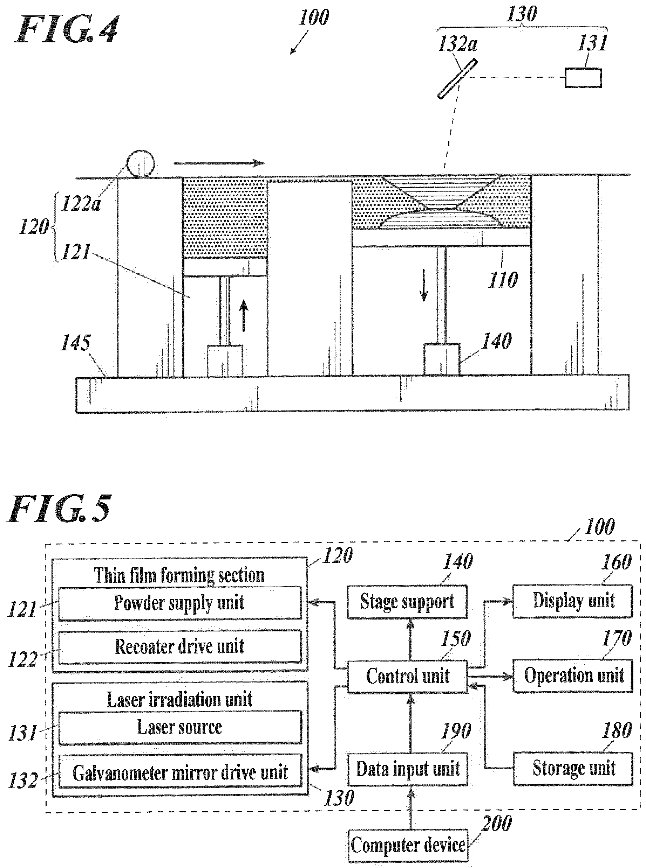

Specifically, the three-dimensional molding apparatus 100 is provided with, as described in FIG. 4, which is a side view schematically showing the configuration thereof, a molding stage 110 located within an aperture, a thin film forming section 120 for forming a thin film of the resin powder for a 3D printer including the resin particles on the above molding stage, a laser irradiation unit 130 that irradiates the thin film with a laser to form a molded object layer containing the above resin particles melt-bonded together, and a stage support 140 that supports the molding stage 110 in a vertical direction at a variable position, and a base 145 supporting each of the above-described parts.

The three-dimensional molding apparatus 100 may be provided with the following as described in FIG. 5, which shows the main part of its control system: a control unit 150 that controls the thin film forming section 120, the laser irradiation unit 130, and the stage support 140 to repeatedly form and stack the molded object layers, a display unit 160 for displaying various information, and an instruction from the user, an operation unit 170 including a pointing device for receiving instructions from a user, a storage unit 180 storing various information including control programs executed by the control unit 150, and a data input unit 190 including an interface for transmitting and receiving various information such as three-dimensional molding data to and from an external device. A computer device 200 for generating data for a 3D printer may be connected to the three-dimensional molding apparatus 100.

On the molding stage 110, a molding material layer is formed by forming a thin layer by the thin film forming section 120 and by irradiating a laser by the laser irradiating unit 130. By stacking these layers of molding material, a three-dimensional molded object is formed.

The thin film forming section 120 may be configured to includes, for example, an opening whose edge is substantially on the same plane in the horizontal direction as the edge of the opening in which the molding stage 110 moves up and down, a powder material storage unit extending vertically downward from the opening, a powder supply unit 121 provided at the bottom of the material storage unit and having a supply piston that moves up and down within the opening, and a recoater 122a that spreads the supplied powder material evenly on the molding stage 110 to form a thin layer of the powder material.

The powder supply section 121 may include a powder material storage unit provided vertically above the molding stage 110 and a nozzle. This configuration allows to discharge the resin powder for a 3D printer on the same plane in the horizontal direction as the molding stage.

The laser irradiation unit 130 includes a laser source 131 and a galvanometer mirror 132a. The laser irradiation unit 130 may be provided with a lens (not shown) to align the focal distance of the laser to the surface of the thin layer. The laser source 131 may be any light source that emits a laser of the above wavelengths at the above output power. Examples of the laser source 131 include YAG laser sources, fiber laser sources and CO₂ laser sources. The galvanometer mirror 132a may be composed of an X mirror that reflects the laser emitted from the laser source 131 and scans the laser in the X direction and a Y mirror that scans the laser in the Y direction.

The stage support 140 supports the molding stage 110 in a variable vertical position thereof. In other words, the molding stage 110 is configured to be precisely movable in the vertical direction by the stage support 140. Various configurations may be employed for the stage support 140. For example, the stage support 140 comprises a holding member that holds the molding stage 110, a guide member that guides the holding member in the vertical direction, and a ball screw engaged with a screw hole provided in the guide member.

The control unit 150 controls the operation of the entire three-dimensional molding apparatus 100 during the molding operation of the orthosis.

The control unit 150 also includes a hardware processor such as a central processing unit. It may be configured that the data input unit 190 converts the three-dimensional molding data acquired from the computer device 200 into a plurality of thinly sliced slice data about the stacking direction of the molding material layers. The slice data is molding data for each molding material layer for molding the orthosis. The thickness of the slice data, i.e., the thickness of the molding material layer, corresponds to a distance (stacking pitch) corresponding to the thickness of one layer of the molding material layer.

The display 160 may be, for example, a liquid crystal display or a monitor.

The operation unit 170 may include a pointing device, such as a keyboard or a mouse, and it may be provided with various operation keys, such as a numeric keypad, an execution key, and a start key.

The storage unit 180 may include, for example, ROM, RAM, magnetic disk, HDD, and SSD.

The three-dimensional molding apparatus 100 may be provided with a pressure reducing section (not shown) such as a pressure reducing pump that reduces the pressure inside the apparatus under the control of the control unit 150, or it may be provided with an inert gas supply unit (not shown) that supplies inert gas into the apparatus under control of the control unit 150. The three-dimensional molding apparatus 100 may also be provided with a heater (not shown) that heats the top surface of the thin layer of the resin powder for a 3D printer under the control of the control unit 150.

### <Example of three-dimensional molding using the three-dimensional molding apparatus 100>

The control unit 150 converts the three-dimensional molding data acquired from the computer device 200 by the data input unit 190 into a plurality of slice data obtained by slicing the molding material layers in the stacking direction. After that, the control unit 150 controls the following operations in the three-dimensional molding apparatus 100.

The powder supply unit 121 drives a motor and a drive mechanism (both not shown) according to the supply information output from the control unit 150, moves the supply piston vertically upward (in the direction of the arrow in the figure), and moves to the molding stage. It extrudes the resin powder for a 3D printer on the same horizontal plane as the molding stage above.

Thereafter, the recoater drive unit 122 moves the recoater 122a in the horizontal direction (arrow direction in the figure) according to the thin film formation information output from the control unit 150, conveys the resin powder for a 3D printer to the molding stage 110, and the powder material is pressed so that the thickness of the thin layer becomes the thickness of one layer of the molded object layer.

After that, according to the laser irradiation information output from the control unit 150, the laser irradiation unit 130 emits a laser from the laser source 131 in conformity with the area constituting the three-dimensional object in each slice data on the thin film, and the galvanometer mirror drive unit 132 drives the galvanometer mirror 132a to scan the laser. The laser irradiation melts and bonds polypropylene resin particles, for example, contained in the resin powder for a 3D printer, to form a molded object layer.

Thereafter, the stage support 140 drives the motor and the drive mechanism (both not shown in the figure) in accordance with the position control information output from the control unit 150 to move the molding stage 110 vertically downward (in the arrow direction in the figure) by the stacking pitch.

The display unit 160 displays various information and messages to be recognized by the user, as necessary, under the control of the control unit 150. The operation unit 170 accepts various input operations by the user and outputs operation signals corresponding to the input operations to the control unit 150. For example, a virtual orthosis to be formed is displayed on the display 160 to check whether the desired shape is formed, and if the desired shape is not formed, the operation unit 170 may be used to make modifications.

The control unit 150 stores data in the storage unit 180 or withdraws data from the storage unit 180, as necessary.

By repeating these operations, the molded object layers are stacked, and the orthosis (three-dimensional molded object) is fabricated.

### EXAMPLES

Hereinafter, the present invention will be specifically described with referring to examples, however, the present invention is not limited thereto. In the examples, "part" or "%" is used to indicate "part by mass" or "mass%" unless otherwise specified.

The following resin materials (resin powder) for a 3D printer were used to make the orthosis.

### [Preparation of Resin powder 1]

As a thermoplastic resin, DuraForm PA (Nylon-based resin material, manufactured by 3D Systems Inc.) was used as Resin powder 1.

### [Preparation of Resin powder 2]

A resin powder was prepared according to the following method.

### (Mechanical pulverization of resin materials)

A polypropylene resin (NOBREN FLX80E4, manufactured by Sumitomo Chemical Co., Ltd.) was used as a crystalline thermoplastic resin. The polypropylene resin was cooled to about -150° C with liquid nitrogen and pulverized with a milling machine (Linlex mill) until the volume average particle diameter became 80 µm. Thus, a polypropylene resin powder was prepared.

### (Particle spheronization treatment)

The polypropylene resin particles prepared by pulverization by the above method were subjected to particle spheroidization treatment according to the following method to prepare a polypropylene resin powder.

### <COMPOSI method>

The pulverized polypropylene resin particles were subjected to particle spheroidizing treatment using COMPOSI CP15 manufactured by Nippon Coke Company.

The spheronization conditions were as follows: a charging amount of 1000 g, a processing time of 45 minutes, and a peripheral velocity of 60 m/s. By this, a resin powder was obtained.

### (Measurement of each particle size characteristic and particle size distribution characteristic value in resin powder)

A volume average particle diameter MV and a number average particle diameter MN of the resin powder were measured with a particle size distribution analyzer (Microtrac MT3300EXII, manufactured by MicrotracBEL Corp.). The particle refractive index of the resin powder was set to 1.5.

The measurement procedure was as follows. 0.1 g of resin powder was weighed, to this were added 0.2 g of surfactant (EMAL E-27C, Kao Corporation) and 30 mL of water. After preparing a sample by ultrasonic dispersion for 10 minutes, the volume average particle diameter MV and the number average particle diameter MN were measured using the above particle size distribution analyzer. The results showed that the volume average particle diameter MV was 80 µm, the number average particle diameter MN was 3.0 µm, and MVMIN was 2.7. In addition, the minimum particle diameter in terms of volume particle diameter was 18 µm, and the maximum particle diameter was 160 µm.

In the resin powder, the number of resin particles having an average particle diameter in the range of 0.15 to 0.41 times the number average particle diameter MN was equal to or greater than the number of particles having the number average particle diameter MN.

### (Measurement of static bulk density)

Using a commercially available bulk density measuring apparatus, a cubic cup is used as the measuring container, the minimum volume is 25 cm³, and the resin powder is poured through the measuring apparatus until the excess powder overflows into the cup that serves as the receiver.
The blade of the spatula, which is placed in vertical contact with the top of the cup, is moved smoothly to carefully scrape off the excess resin powder from the top of the cup, keeping the spatula vertical to prevent compaction and overflow of the powder from the cup. All the sample is then removed from the sides of the cup as well, and the mass of the powder (m) is measured to 0.1 %.

Then, by the formula: m/V₀ (V₀ is the volume of the cup), the static bulk density (g/cm³) for the three different samples, and the average value was determined to be 0.370 (g/cm³).

Resin powder 2 was prepared by adding 0.7 mass% silica particles (AEROSIL R972, average particle diameter: 16, manufactured by NIPPON AEROSIL Co., Ltd.) as an inorganic oxide to the resin powder obtained above.

### [Preparation of Resin powder 3]

Resin powder 3 was prepared in the same manner as Resin powder 2, except that the amount of silica particles in resin powder 2 was 0.3 mass%.

### [Preparation of Resin powder 4]

Resin Powder 4 was prepared in the same manner as Resin powder 2, except that the amount of silica particles in resin powder 2 was 0.28 mass%.

### [Preparation of Resin powder 5]

As a crystalline thermoplastic resin, a polypropylene resin (NOBREN FLX80E4, manufactured by Sumitomo Chemical Co., Ltd.) was mixed with 1 mass% of talc particles (Micron White #5000, manufactured by Hayashi Kasei Co., Ltd.,). The mixture was kneaded in a small kneading machine (MC15, made by Xplore Co., Ltd.). The resulting mixture was cooled to about -150 °C with liquid nitrogen and pulverized with a milling machine (LINREX Mill) until the volume average particle diameter became 80 µm. The particles were spheroidized in the same manner as resin powder 2, and 0.2 mass% silica particles were added to prepare Resin powder 5.

### [Preparation of Resin powder 6]

Resin powder 6 was prepared in the same manner as Resin powder 5, except that the amount of silica particles in resin powder 5 was reduced to 0.1 mass%, and of talc particles were added in an amount of 1.5 mass%.

### [Preparation of Resin powder 7]

Resin powder 7 was prepared in the same manner as Resin powder 6, except that the polypropylene resin of resin powder 6 was changed to PMA 20V made by SunAllomer Ltd.

### [Preparation of Resin powder 8]

Resin powder 8 was prepared in the same manner as Resin powder 6, except that the amount of the talc particles in resin powder 6 were changed to 5 mass%.

### [Fabrication of Orthoses 1 to 8]

A three-dimensional molding apparatus sPro 140 (manufactured by 3D Systems Inc.) was used. The above-mentioned resin powders in particle form were spread on the molding stage at a specified recoat speed (100 mm/s) to form a thin layer of 0.1 mm thick.

Under the following conditions, this thin layer was irradiated with a laser beam from a CO₂ laser equipped with a YAG wavelength galvanometer scanner in an area of 15 mm long x 20 mm wide under the emission conditions and scanning conditions described below to form a molded object layer. Thereafter, the resin powder was further spread on the molded object layer, irradiated with laser beams, and the molded layer was stacked. These processes were repeated to fabricate a three-dimensionally stacked molded object.

### (Laser beam emission conditions)

Laser power: 12 W
Laser beam wavelength : 10.6 µm
Beam diameter: 170 µm on thin layer surface

### (Laser beam scanning conditions)

Scanning speed: 2000 mm/sec
Number of lines : 1 line

### «Evaluation»

The test piece for evaluating the following elongation at break and tensile modulus are prepared as follows.

### <Production of Injection-molded object>

The resin materials (resin powders 1 to 8) for a 3D printer prepared above were used.

Shape of test piece: The shape was adjusted to conform to JIS-K716:2014:2014.

### (Injection molding conditions)

Mixing temperature: Melting point +30 °C
Injection pressure: 10 MPa
Molding machine: Injection molding machine, manufactured by Leo Labs, Xplore Instruments Inc.
Reheating and slow cooling conditions: N₂ Oven, Raise the temperature to "the melting point -10 °C" and then cool down to 50 °C for 7 hrs.

### <Elongation at break>

The elongation at break was measured using a universal material testing machine TENSILON RTC-1250 (manufactured by A & D Co., Ltd.) to the obtained test pieces. The measurement conditions were set as follows. The distance to fracture was made as the elongation at break, which was evaluated based on the presence or absence of a yield point. (If the material was fractured before the yield point, it was assumed to have no yield point).

Test piece for tensile test: Shape to comply with JIS K7161: 2014: 2014: 2014
Tensile speed: 1 mm/min
Distance between chucks: 115 mm
Gauge distance: 75 mm

### <Tensile modulus>

The tensile modulus was measured to the obtained test pieces with a universal material testing machine TENSILON RTC-1250 (manufactured by A & D Co., Ltd.). The measurement conditions were set as follows, and the tensile modulus was obtained by linear regression between 0.05 and 0.25% strain. In addition, when a value of 80% or more (that is, a value of 1160 Mpa or more) was shown with respect to the tensile modulus (1450 Mpa) of Comparative Example 1, it was regarded having no problem for practical use.

Test piece for tensile test: Shape to comply with JIS K7161: 2014: 2014
Tensile speed: 1 mm/min
Distance between chucks: 115 mm
Gauge distance: 75 mm

### <Evaluation 1: Breaking test of orthosis>

The presence or absence of breakage of the orthosis at dorsiflexion 20° was evaluated.

The presence or absence of breakage at 20° dorsiflexion was evaluated using a rigidity measurement evaluation machine (self-made).

The plantar part of the orthosis to be worn on the lower leg was fixed, and the lower leg part of the orthosis was moved in the dorsiflexion direction with the outer ankle as the center, and the orthosis was deformed at an angle of ±20° around the ankle (refer to FIG. 3A).

Double circle: No breakage at all, there is no change.

Circle: Slight traces of breakage are observed.

Triangle: Traces of breakage are observed.

Cross mark: Breakage is observed.

Double circle and Circle are acceptable are acceptable for practical use.

### <Evaluation 2: Evaluation of thickness of orthosis (Comfort)>

Using the above-produced orthotic, the rigidity was measured using a rigidity measurement evaluation machine (manufactured by ourselves) (refer to FIG. 3A and FIG. 3B.). The plantar part of the orthosis to be worn on the lower leg was fixed, and the lower leg part of the orthosis was moved in the plantar and dorsiflexion direction by a rotating shaft with the ankle at the center, and the reaction force moment was measured for each 1° angle in the plantar and dorsiflexion direction.

The measurement data measures the reaction force moment every 1° in the plantar and dorsiflexion direction, and the measurement angle range assumes hemiplegic walking, for example, from dorsiflexion 8° to plantar flexion 8°.

The data were plotted on the coordinates of the reaction force moment [Nm] on the vertical axis and the plantar and dorsiflexion angle [deg] on the horizontal axis. A hysteresis curve was obtained, and the slope of the approximation curve near the angle of 0° was defined as the rigidity of the orthosis [Nm/deg].

Next, samples were prepared by changing the type of resin material used for the orthosis and the thickness of the orthosis, and the thickness of the orthosis at which the "rigidity" reached 1 Nm/deg was determined. By this, the thickness of the orthosis to be practical and comfortable was determined.

The obtained orthotic thicknesses were ranked as follows.
Double circle: 3 mm or less
Circle: Over 3 mm to 3.5 mm or less
Triangle: Over 3.5 mm to 4.5 mm or less
Cross mark: Over 4.5 mm

From the standpoint of weight reduction and wearability of the orthosis, Triangle, Circle, and Double circle are acceptable for practical use, and Circle or Double circle is preferable from the viewpoint of comfort.

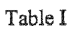

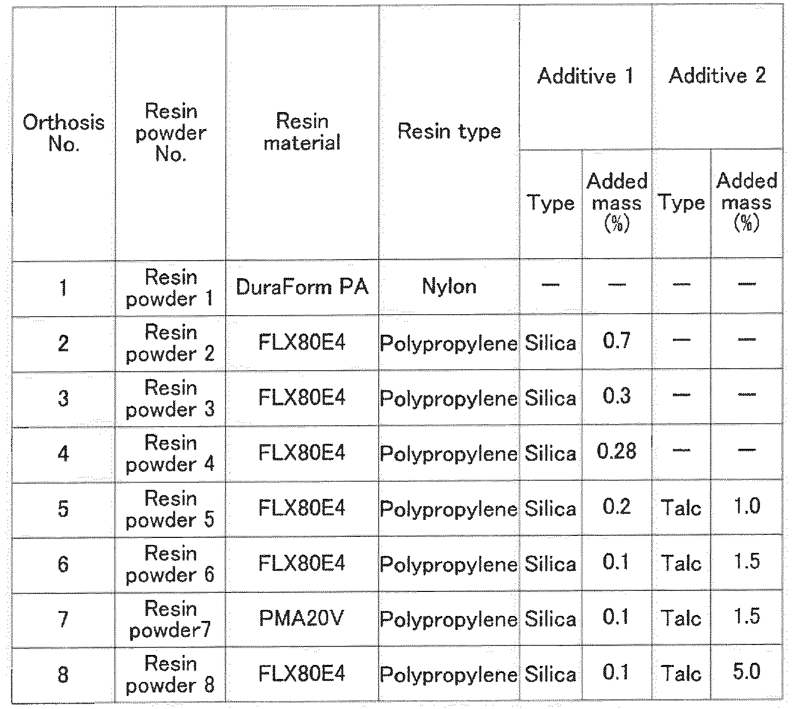

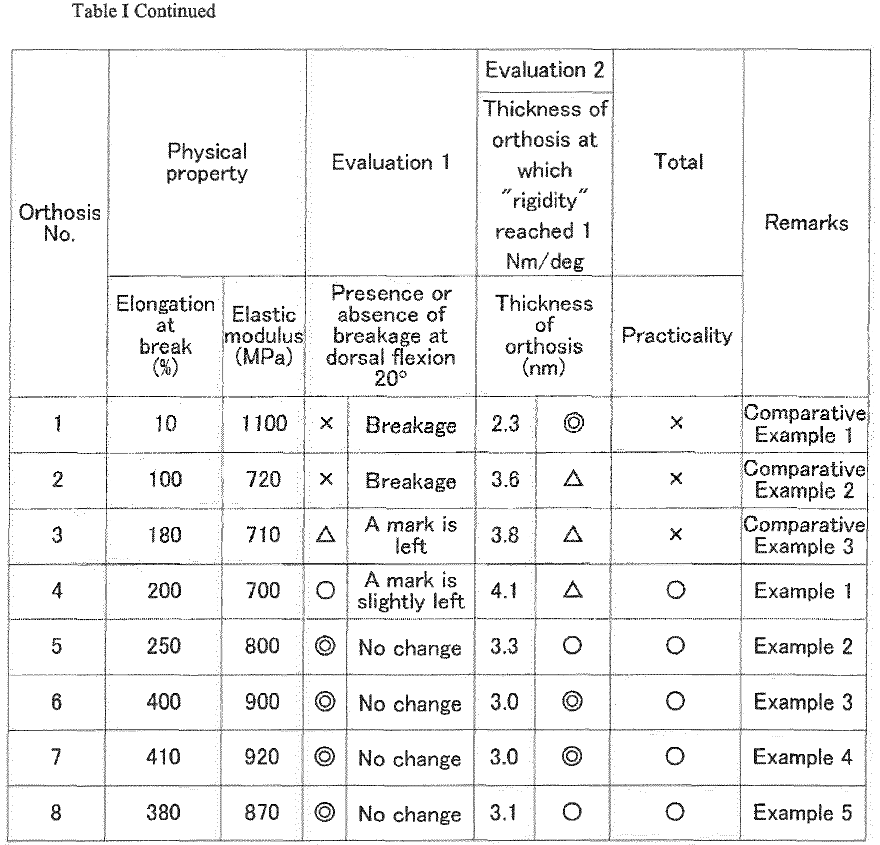

From Table I, the materials with an elongation at break less than 200% was damaged during the evaluation of orthotic rigidity. The material with an elongation at break greater than 200% was not damaged. This indicates that when the resin material with a tensile rupture elongation greater than 200% was used in the orthosis, it was confirmed to be a body-mounted component having excellent flexibility and suppressing the occurrence of breakage when flexed, since the occurrence of breakage at dorsiflexion 20° was suppressed. In addition, the orthosis of the present invention was comfortable to wear because of its thin thickness, and had excellent rigidity, and thus it was an orthosis with excellent practicality.

### INDUSTRIAL APPLICABILITY

The body-mounted component of the present invention is a body-mounted component molded by a 3D printer, and has excellent flexibility, suppresses the occurrence of breakage when it is bent, and has comfortable wearability, making it suitable for use as a body-mounted component used around a body joint in particular.

### REFERENCE SIGNS LIST

- 1:: Human body shape measurement unit
- 2:: Orthosis molding data generating unit
- 3:: Orthosis molding unit (warning section)
- 4:: Orthosis shape measuring unit (data completion unit, display unit, error display unit)
- 5:: Management DB
- A:: Orthosis manufacturing system
- M:: Orthosis (short leg orthosis)
- Mt:: Band
- T:: Post-processing of orthosis by orthotist
- K:: Drive portion
- Rs:: Rotating shaft
- Pa:: Plane stand
- 100:: Three-dimensional molding apparatus
- 110:: Molding stage
- 120:: Thin film forming section
- 121:: Powder supply unit
- 122:: Recoater drive unit
- 122a:: Recoater
- 130:: Laser irradiation unit
- 131:: Laser source
- 132:: Galvanometer mirror drive unit
- 132a:: Galvanometer mirror
- 140:: Stage support
- 145:: Base
- 150:: Control unit
- 160:: Display unit
- 170:: Operation unit
- 180:: Storage unit
- 190:: Data input unit
- 200:: Computer device

## Claims

1. A body-mounted component to be worn around a joint of a body, wherein the body-mounted component is formed from a resin material for a 3D printer; and a tensile rupture elongation of the resin material is 200% or more.

2. The body-mounted component according to claim 1, wherein a tensile modulus of the resin material is 800 MPa or more.

3. The body-mounted component according to claim 1 or 2, wherein the tensile rupture elongation is 400% or more, and the tensile modulus is 900 MPa or more.

4. The body-mounted component according to any one of claims 1 to 3, wherein the resin material is polypropylene.

5. The body-mounted component according to any one of claims 1 to 4, wherein the resin material contains talc as an additive in the range of 1.0 to 5.0 mass%.

6. A method for manufacturing a body-mounted component according to any one of claims 1 to 5, comprising:
a process 1 of forming a thin layer of a powder of the resin material for a 3D printer;
a process 2 of selectively irradiating a laser beam onto the formed thin layer to form a molded object layer in which resin particles contained in the powder are sintered or melt-bonded; and
a process 3 of repeating the process 1 of forming the thin layer and the process 2 of forming the molded object layer in this order a plurality of times to laminate the molded object layers.
